Europäisches Patentamt

European Patent Office

Office européen des brevets

(19)

(11) Veröffentlichungsnummer: **0 249 617**
**B1**

(12) **EUROPÄISCHE PATENTSCHRIFT**

(45) Veröffentlichungstag der Patentschrift: **18.04.90**

(21) Anmeldenummer: **87900099.0**

(22) Anmeldetag: **03.12.86**

(86) Internationale Anmeldenummer:
**PCT/EP86/00701**

(87) Internationale Veröffentlichungsnummer:
**WO 87/03493 18.06.87 Gazette 87/13**

(51) Int. Cl.⁵: **A 61 M 5/14, F 16 K 7/04**

(54) **REGULIERKLEMME FÜR INFUSIONSSCHLÄUCHE.**

(30) Priorität: **04.12.85 DE 3542899**

(43) Veröffentlichungstag der Anmeldung:
**23.12.87 Patentblatt 87/52**

(45) Bekanntmachung des Hinweises auf die
Patenterteilung:
**18.04.90 Patentblatt 90/16**

(84) Benannte Vertragsstaaten:
**AT BE CH FR GB IT LI NL SE**

(56) Entgegenhaltungen:
**US-A-3 254 869**
**US-A-3 818 945**
**US-A-4 047 694**

(73) Patentinhaber: **Pfrimmer-Viggo GmbH + Co. KG**
**Langemarckplatz 3 Postfach 28 80**
**D-8520 Erlangen (DE)**

(72) Erfinder: **IWATSCHENKO, Peter**
**Bürgerholzweg 4**
**D-8524 Neunkirchen (DE)**
Erfinder: **WOLKENSTÖRFER, Reinhold**
**Anna-Friedrich-Strasse 5a**
**D-8524 Neunkirchen (DE)**
Erfinder: **HOFMANN, Wolfgang**
**Hollergasse 12**
**D-8551 Heroldsbach (DE)**
Erfinder: **PRELL, Walter**
**Brunnenweg 1**
**D-8551 Hallerndorf (DE)**

(74) Vertreter: **von Hellfeld, Axel, Dipl.-Phys. Dr. et al**
**Wuesthoff & Wuesthoff Patent- und**
**Rechtsanwälte Schweigerstrasse 2**
**D-8000 München 90 (DE)**

EP 0 249 617 B1

Courier Press, Leamington Spa, England.

**Beschreibung**

Die Erfindung betrifft eine Regulierklemme zum Steuern der Durchflußrate einer Flüssigkeit durch einen flexiblen Schlauch, wie den Schlauch eines Infusionsgerätes, mit einer Einrichtung zum Ändern des Strömungsquerschnittes des Schlauches.

Derartige Regulierklemmen sind z.B. aus der DE—OS 20 43 551, der DE—PS 22 42 539, der DE—OS 27 41 594 und der US—PS 32 15 395 bekannt.

Für die parenterale Verabreichung von Flüssigkeiten mittels Infusions- oder Transfusionsgeräten haben sich für den einmaligen Gebrauch vorgesehene Packungen weitgehend durchgesetzt. Solche Packungen enthalten üblicherweise einen Schlauch aus flexiblem Material, wie PVC (Polyvenylchlorid), sowie eine Regulierklemme, mittels derer der Strömungsquerschnitt des Schlauches veränderbar ist, um die Fließgeschwindigkeit der Flüssigkeit nach Wunsch einzustellen. Typische Fließgeschwindigkeiten sind 50 bis 120 ml/Std, was etwa 25 bis 60 Tropfen/Min entspricht. Bei bekannten Regulierklemmen werden solche Fließgeschwindigkeiten dadurch eingestellt, daß der Schlauch mittels der Regulierklemme bis auf eine Spaltbreite von etwa 4/100 mm zusammengequetscht wird.

Ist die Fließgeschwindigkeit der parenteral einzugebenden Flüssigkeit einmal eingestellt, so soll sie sich möglichst nicht mehr unwillkürlich ändern. Die hier in Rede stehenden Schläuche, insbesondere aus PVC, haben aber bekanntlich die Eigenschaft, sich unter Druck plastisch zu verformen. Dieser sogenannte "Kaltfluß" des Kunststoffes bewirkt langfristig eine Änderung des Strömungsquerschnittes, so daß die geforderte Konstanz der Fließgeschwindigkeit nicht gewährleistet ist.

Bereits die DE—OS 20 43 541 stellt sich die Aufgabe, eine Regulierklemme zu schaffen, bei der das Kaltfließen verhindert ist. Daß dieses Ziel dort nicht vollständig erreicht ist, bestätigt der gleiche Anmelder in seiner jüngeren DE—OS 26 53 515, S. 4, Zeile 6.

In der DE—PS 22 42 539 soll der Kaltfluß des Schlauchmaterials dadurch verhindert werden, daß asymmetrisch auf einer Seite einer Klemmfläche eine Aussparung vorgesehen ist, in die ein sogenannter Überhang des Schlauches beim Quetschen gebildet wird, welcher auch im Klemmzustand weitgehend frei von seine Form verändernden mechanischen Kräften ist und deshalb keinen Kaltfluß aufweisen soll.

Die Fließgeschwindigkeit der parenteral eingegebenen Flüssigkeit wird außer durch den Strömungsquerschnitt des Schlauches auch durch andere Parameter, wie die beidseitig des Schlauches wirkenden Druckverhältnisse bestimmt. Kritisch sind dabei insbesondere patientenseitige Druckänderungen, durch welche die Strömungsverhältnisse unerwünscht verändert werden können. Die bekannten Regulierklemmen steuern den Strömungsquerschnitt des Schlauches im wesentlichen nur in einem eng begrenzten Abschnitt. Diese "Punktwirkung" der bekannten Regulierklemmen hat zur Folge, daß patientenseitige Druckänderungen ohne Dämpfung die Strömungsverhältnisse unmittelbar ändern und somit die Fließgeschwindigkeit unerwünscht schwankt.

Die Beschränkung der Klemmwirkung auf einen relativ kurzen Abschnitt des Schlauches hat auch nachteilig zur Folge, daß der Strömungsquerschnitt durch Zugkräfte in Längsrichtung des Schlauches verändert werden kann.

In der US—PS 32 15 395 wird eine Regulierklemme beschrieben, mittels derer der Schlauch über eine gewisse Wegstrecke abklemmbar ist, doch ist die Länge der Wegstrecke dort durch den Umfang einer vieleckig ausgeformten Klemmrolle begrenzt. Auch verlangt die dort beschriebene Klemmrolle einiges Geschick seitens der Bedienungsperson und verhindert offensichtlich den Kaltfluß nicht.

Der Erfindung liegt die Aufgabe zugrunde, eine gattungsgemäße Regulierklemme derart auszubilden, daß bei Verhinderung des Kaltflusses die Fließgeschwindigkeit der Flüssigkeit durch den flexiblen Schlauch nach ihrer Einstellung konstant und auch durch patientenseitige Druckschwankungen weitgehend unbeeinflußt bleibt.

Erfindungsgemäß wird diese Aufgabe dadurch gelöst, daß die Regulierklemme mit einer Einrichtung zum Ändern des Strömungsquerschnittes versehen ist, die zumindest drei den Schlauch zwischen sich einklemmende Klemmbacken aufweist, deren relativer Abstand veränderbar ist.

Während im Stand der Technik jeweils nur zwei Klemmteile gegeneinander verschoben werden, sind erfindungsgemäß zumindest und vorzugsweise drei den Schlauch zwischen sich einklemmende Klemmbacken vorgesehen.

Der Abstand der drei Klemmbacken ist zur Veränderung des Strömungsquerschnittes von Hand einstellbar.

In einer bevorzugten Ausgestaltung der Erfindung sind die drei Klemmbacken im Schnitt senkrecht zur Längsachse des Schlauches zumindest annähernd kreissektorförmig und weisen jeweils die gleiche Form auf.

Die drei kreissektorförmigen Klemmbacken sind in einer bevorzugten Ausgestaltung der Erfindung derart entlang von die Längsachse des Schlauches schneidenden Radien verschiebbar, daß die zentralen Kanten der Klemmbacken auf den drei Radien in Richtung auf die Längsachse des Schlauches bewegbar sind und so den Schlauch seitlich in Freiräume zwischen den Klemmbacken einquetschen, wodurch der Schlauch derart verformt wird, daß sich die Schlauchwände großteils planparallel aneinanderlegen und der freibleibende Strömungsquerschnitt zumindest annähernd die Form eines Dreieckes mit konkav gekrümmten Seiten erhält.

Durch die erfindungsgemäße Regulierklemme läßt sich ein relativ langer Abschnitt des Schlauches gleichmäßig verengen, da die Klemmbacken den Schlauch über eine längere Strecke, wie beispielsweise 2 bis 5 cm, einklemmen können.

Im Querschnitt ergibt sich somit ein rotationssymmetrischer Klemmzustand des Schlauches, wobei diejenigen Abschnitte der Schlauchwand, welche starken Biege- und Verformungskräften ausgesetzt sind, weit von denjenigen Wandabschnitten entfernt sind, welche den Strömungsquerschnitt definieren. Letztere Wandabschnitte werden deshalb auch im Klemmzustand ihre Form nicht verändern, so daß der Strömungsquerschnitt konstant bleibt.

Die kreissektorförmigen Klemmbacken bilden in einer vorteilhaften Weiterbildung der Erfindung einen Kreiszylinder oder Konus, so daß sie mittels einer äußeren Hülse zusammendrückbar sind.

Hierzu sind die Klemmbacken vorzugsweise mit einem Außengewinde und die Hülse mit einem Innengewinde versehen, wobei die Hülse und die Klemmbacken gegenläufig konisch geformt sind.

Es ist auch möglich, die Klemmbacken mittels eines Schlauchbinders symmetrisch zusammenzudrücken.

Damit der Schlauch in bezug auf seine Längsachse rotationssymmetrisch (bei drei Klemmbacken mit einem Drehwinkel von 120°) zwischen den Klemmbacken eingequetscht wird und dabei in den Freiräumen zwischen den Klemmbacken jeweils gleiche Schlauchabschnitte eingeklemmt werden, ist in einer bevorzugten Weiterbildung der Erfindung ein Zentriereinsatz zwischen den Klemmbacken vorgesehen.

Auch kann der Schlauch zur Zentrierung dreieckförmig vorgeformt sein.

Es ist auch möglich, den relativen Abstand der Klemmbacken mittels einer Kulissenführung, wie sie bei herkömmlichen Regulierklemmen gebräuchlich sind, einzustellen.

Nachfolgend ist ein Ausführungsbeispiel der Erfindung anhand der Zeichnung näher erläutert. Es zeigt:

Fig. 1 einen Schnitt durch die Klemmbacken und den eingeklemmten Schlauch und

Fig. 2 einen Schnitt entlang der Linie A—B der Fig. 1.

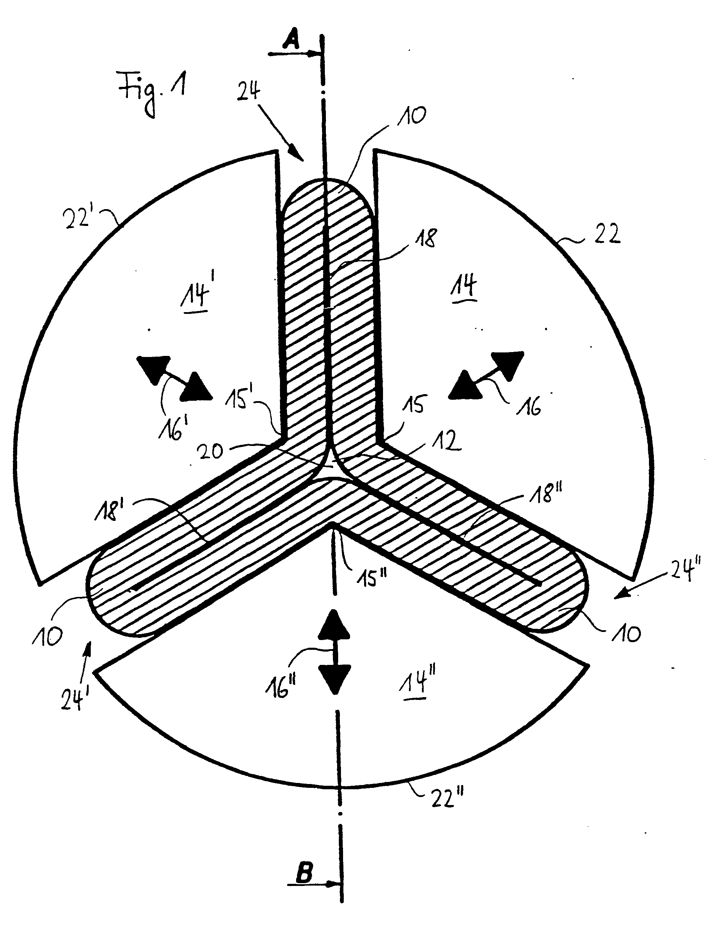

Gemäß Fig. 1 wird der Schlauch 10 aus PVC mittels der Klemmbacken 14, 14', 14'' auf den Strömungsquerschnitt 12 verengt. Hierzu sind die Klemmbacken jeweils entlang der Radien 16, 16', 16'' in Richtung der Pfeile verschiebbar. Die zentralen Kanten 15, 15', 15'' der kreissektorförmigen Klemmbacken 14, 14', 14'' bewegen sich auf den Radien 16, 16', 16'', so daß der Schlauch 10 in einen um seine Längsachse 20 rotationssymmetrischen Klemmzustand überführt wird. Gemäß Fig. 1 liegen dabei die Innenwände des Schlauches 10 entlang der Anlageflächen 18, 18', 18'' weitgehend planparallel dicht aneinander, so daß nur der im Schnitt dreieckförmige Strömungsquerschnitt 12 mit konkaven Seitenflächen freibleibt.

Die Umfangsabschnitte 22, 22', 22'' der Klemmbacken 14, 14', 14'' bilden zumindest annähernd einen Zylinder oder Konus. Zwischen den Klemmbacken bleiben gemäß Fig. 1 Freiräume 24, 24', 24'', in denen der Schlauch 10 jeweils teilweise abgeklemmt ist.

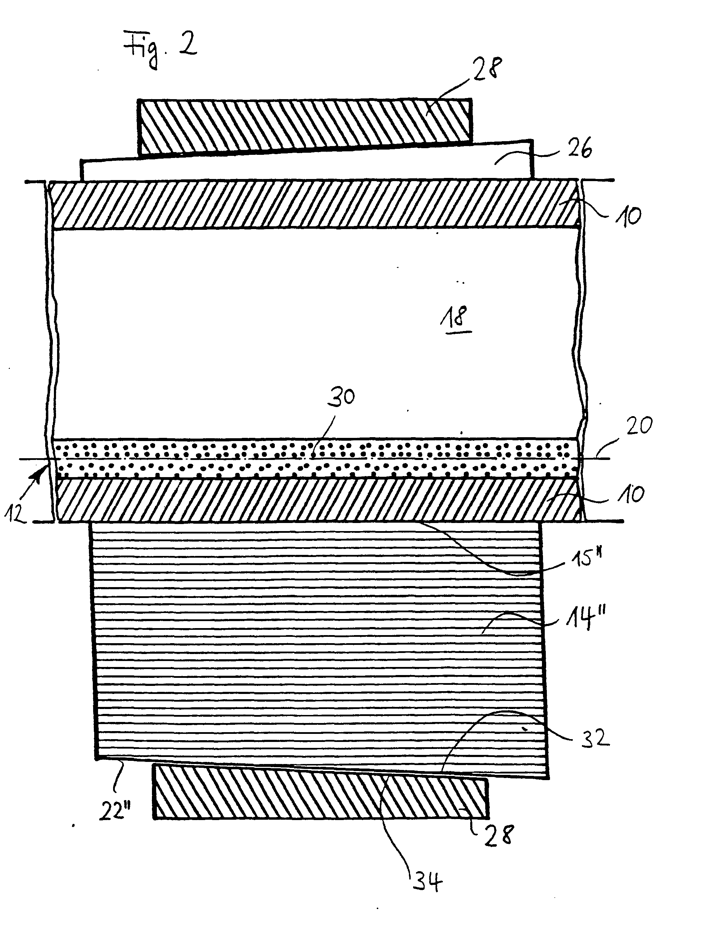

Fig. 2 zeigt einen Schnitt entlang der Linie A—B der Fig. 1. Zusätzlich zu den in Fig. 1 gezeigten Bauteilen sind noch ein Zentriereinsatz 26 und eine äußere Hülse 28 vorgesehen. Der Zentriereinsatz 26 ist jeweils in den Freiräumen 24, 24', 24'' zwischen den Klemmbacken vorgesehen und bewirkt, daß jeweils gleichlange Abschnitte des Schlauches 10 in den Freiräumen 24, 24', 24'' abgeklemmt werden. Die Zentriereinsätze 26 schließen außenseitig bündig mit den Umfangsabschnitten 22, 22', 22'' der Klemmbacken ab und ergänzen diese zumindest annähernd zu einem vollen Kreis (nicht gezeigt).

Auf den Zentriereinsatz kann insbesondere dann verzichtet werden, wenn der Schlauch 10 zumindest im Bereich der Klemmbacken 14, 14' und 14'' derart vorgeformt ist, daß die Kanten 15, 15', 15'' der Klemmbacken zwangsläufig rotationssymmetrisch mit einem Drehwinkel von 120° am Schlauch 10 angreifen. Hierzu ist der Schlauch 10 von vorneherein im Bereich der Regulierklemme im Schnitt etwa dreieckförmig plastisch vorgeformt und weist drei sich parallel zur Längsachse 20 erstreckende Führungsrillen (nicht gezeigt) auf, in welche die spitzen Kanten 15, 15', 15'' der Klemmbacken derart eingreifen, daß in den Freiräumen 24, 24', 24'' jeweils gleiche Schlauchabschnitte abgeklemmt werden.

Im in Fig. 2 gezeigten Ausführungsbeispiel werden die Klemmbacken 14, 14', 14'' entlang der durch die Längsachse 20 des Schlauches 10 gehenden Radien 16, 16', 16'' mittels einer Hülse 28 bewegt. Hierfür sind die äußeren Umfangsabschnitte 22, 22', 22'' der Klemmbacken mit einem Außengewinde 32 und der Innenumfang der Hülse 28 mit einem Innengewinde 34 versehen. Je nach dem Schraubzustand zwischen Hülse und Klemmbacken wird somit der Strömungsquerschnitt 12 des Schlauches 10 verändert und die Fließgeschwindigkeit der Flüssigkeit 30 gesteuert.

Gemäß Fig. 2 erstrecken sich die spitzen Kanten 15, 15', 15'' der Klemmbacken zumindest annähernd parallel zur Längsachse 20 des Schlauches 10. Somit kann ein relativ langer Schlauchabschnitt von z.B. 2 bis 5 cm abgeklemmt werden und die Drosselung der Fließgeschwindigkeit mittels der Regulierklemme weist eine gute Dämpfung auf, d.h. patientenseitige Druckschwankungen werden nur sehr stark gedämpft auf die andere Seite der Regulierklemme übertragen, so daß die Fließgeschwindigkeit bei momentanen patientenseitigen Druckschwankungen nicht sofort vom Sollwert abweicht.

**Patentansprüche**

1. Regulierklemme zum Steuern der Durchflußrate einer Flüssigkeit durch einen flexiblen Schlauch (10), wie den Schlauch eines Infusionsgerätes, mit einer Einrichtung zum Ändern des Strömungsquerschnittes (12) des Schlauches dadurch gekennzeichnet, daß die Einrichtung zum Ändern des Strömungsquerschnittes (12) zumindest drei den Schlauch (10) zwischen sich einklemmende Klemmbacken (14, 14', 14'') aufweist, deren relativer Abstand Veränderbar ist.

2. Regulierklemme nach Anspruch 1, dadurch gekennzeichnet, daß die Einrichtung zum Ändern des Strömungsquerschnittes von Hand betätigbar ist.

3. Regulierklemme nach einem der Ansprüche 1 oder 2, dadurch gekennzeichnet, daß drei den Schlauch (10) zwischen sich einklemmende Klemmbacken (14, 14', 14'') vorgesehen sind.

4. Regulierklemme nach einem der vorhergehenden Ansprüche, dadurch gekennzeichnet, daß die Klemmbacken (14, 14', 14'') im Schnitt senkrecht zur Längsachse (20) des Schlauches (10) zumindest annähernd kreissektorförmig sind.

5. Regulierklemme nach einem der vorhergehenden Ansprüche, dadurch gekennzeichnet, daß die Klemmbacken (14, 14', 14'') gleiche Form aufweisen.

6. Regulierklemme nach einem der vorhergehenden Ansprüche, dadurch gekennzeichnet, daß die Klemmbacken (14, 14', 14'') entlang von Radien (16, 16', 16'') verschiebbar sind, welche die Längsachse (20) des Schlauches (10) schneiden.

7. Regulierklemme nach Anspruch 6, dadurch gekennzeichnet, daß die mit dem Schlauch (10) in Eingriff stehenden Kanten (15' 15', 15'') der Klemmbacken (14, 14', 14'') auf den Radien (16, 16', 16'') liegen.

8. Regulierklemme nach Anspruch 7, dadurch gekennzeichnet, daß die Klemmbacken (14, 14', 14'') beim Verschieben zum Verringern des Strömungsquerschnittes (12) des Schlauches (10) seitlich im Bezug auf ihre Bewegungsrichtung Freiräume (24, 24', 24'') zwischen sich aufweisen, in denen der Schlauch derart verformbar ist, daß die Schlauchwände großteils planparallel aneinanderliegen und der Strömungsquerschnitt zumindest annähernd die Form eines drei zackigen Sternes aufweist.

9. Regulierklemme nach einem der vorhergehenden Ansprüche, dadurch gekennzeichnet, daß die Klemmbacken (14, 14', 14'') den Schlauch (10) zumindest annähernd parallel entlang einer Strecke von 1 bis 50mm klemmen.

10. Regulierklemme nach einem der vorhergehenden Ansprüche zusammen mit einem Schlauch, dadurch gekennzeichnet, daß der Schlauch (10) zumindest im Bereich der Regulierklemme plastisch derart vorgeformt ist, daß er symmetrisch zwischen die Klemmbacken (14, 14', 14'') einklemmbar ist.

11. Regulierklemme nach einem der vorhergehenden Ansprüche, dadurch gekennzeichnet, daß die Außenflächen (22, 22', 22'') der Klemmbacken (14, 14', 14'') zumindest annähernd auf einem Zylinder- oder Konusumfang liegen und mittels einer die Klemmbacken umfaßenden Hülse (28) zusammendrückbar sind.

12. Regulierklemme nach Anspruch 11, dadurch gekennzeichnet, daß die Klemmbacken (14, 14', 14'') mit einem Außengewinde und die Hülse (28) mit einem Innengewinde versehen sind, wobei die Hülse (28) und die Klemmbacken (14, 14', 14'') gegenläufig konisch geformt sind.

13. Regulierklemme nach einem der Ansprüche 1 bis 10, dadurch gekennzeichnet, daß die Klemmbacken (14, 14', 14'') mittels eines Schlauchbinders zusammendrückbar sind.

14. Regulierklemme nach einem der vorhergehenden Ansprüche, dadurch gekennzeichnet, daß zwischen den Klemmbacken (14, 14', 14'') ein Zentriereinsatz (26) für den Schlauch (10) vorgesehen ist, um eine zentrische, symmetrische Führung des Schlauches in Bezug auf die Klemmbacken zu gewährleisten.

15. Regulierklemme nach einem der vorhergehenden Ansprüche, dadurch gekennzeichnet, daß eine Kulissenführung zum Verschieben der Klemmbacken (14, 14', 14'') vorgesehen ist.

**Revendications**

1. Pince de réglage servant à commander le débit d'écoulement d'un liquide dans un tuyau flexible (10) tel que le tuyau d'un appareil de perfusion, comprenant un dispositif permettant de faire varier la section d'écoulement (12) du tuyau, caractérisée en ce que le dispositif permettant de faire varier la section d'écoulement (12) comprend au moins trois mors (14, 14', 14'') qui serrent le tuyau (10) entre eux et dont la distance d'écartement relatif est variable.

2. Pince de réglage selon la revendication 1, caractérisée en ce que le dispositif permettant de faire varier la section d'écoulement peut être manoeuvré à la main.

3. Pince de réglage selon une des revendications 1 et 2, caractérisée en ce qu'il est prévu trois mors (14, 14', 14'') qui serrent le tuyau (10) entre eux.

4. Pince de réglage selon une des revendications précédentes, caractérisée en ce que, considérés dans une section perpendiculaire à l'axe longitudinal (20) du tuyau (101, les mors (14, 14', 14'') sont au moins approximativement en forme de secteur de cercle.

5. Pince de réglage selon une des revendications précédentes, caractérisée en ce que les mors (14, 14', 14'') présentent tous la même forme.

6. Pince de réglage selon une des revendications précédentes, caractérisée en ce que les mors (14, 14', 14'') peuvent se déplacer en translation le long de rayons (16, 16', 16'') qui coupent l'axe longitudinal (20) du tuyau (10).

7. Pince de réglage selon la revendication 6, caractérisée en ce que les arêtes (15, 15', 15'') des mors (14, 14', 14'') qui sont en prise avec le tuyau (10) se trouvent sur les rayons (16, 16', 16'').

8. Pince de réglage selon la revendication 7, caractérisée en ce que, lorsqu'ils se déplacent en translation pour réduire la section d'écoulement (12) du tuyau (10), les mors (14, 14', 14'') présentent entre eux des espaces libres (24, 24', 24'') situés latéralement par rapport à leur directionde déplacement, dans lesquels le tuyau peut se déformer de telle manière que les parois du tuyau soient en majeure partie appliquées l'une contre l'autre dans des dispositions planes parallèles, et la section d'écoulement présente au moins approximativement la forme d'une étoile à trois branches.

9. Pince de réglage selon une des revendications précédentes, caractérisée en ce que les mors (14, 14', 14'') serrent le tuyau (10) au moins approximativement parallèlement sur une distance de 1 à 50 mm.

10. Pince de réglage selon une des revendications précédentes, associée à un tuyau, caractérisée en ce que le tuyau (10) est préformé, au moins dans la région du mors par déformation plastique, de manière qu'il puisse être serré symétriquement entre les mors (14, 14', 14'').

11. Pince de réglage selon une des revendications précédentes, caractérisée en ce que les surfaces externes (22, 22', 22'') des mors (14, 14', 14'') se trouvent au moins approximativement sur une surface périphérique de cylindre ou de cône et peuvent être resserrées les unes contre les autres au moyen d'un manchon (28) qui entoure les mors.

12. Pince de réglage selon la revendication 11, caractérisée en ce que les mors (14, 14', 14'') sont munis d'un filetage extérieur et le manchon (28) d'un filetage intérieur, le manchon (28) et les mors (14, 14', 14'') présentent des formes coniques complémentaires.

13. Pince de réglage selon une des revendications 1 à 10, caractérisée en ce que les mors (14, 14', 14'') peuvent être resserrés les uns contre les autres au moyen d'un collier de tube.

14. Pince de réglage selon une des revendications précédentes, caractérisée en ce qu'il est prévu entre les mors (14, 14', 14'') un insert de centrage (26) servant à centrer le tuyau (10), afin de garantir un guidage symétrique centré du tuyau par rapport aux mors.

15. Pince de réglage selon une des revendications précédentes, caractérisée en ce qu'il est prévu un guidage à coulisse pour la translation des mors (14, 14', 14'').

**Claims**

1. A control clamp for adjusting the flow rate of a liquid through a flexible hose (10), such as the hose of an infusion device, comprising a means for varying the flow cross section (12) of the hose, characterized in that the means for varying the flow cross section (12) comprises at least three clamping jaws 14, 14', 14'') between which the hose (10) is clamped and the relative spacing is variable.

2. The control clamp as claimed in claim 1, characterized in that the means for varying the flow cross section is operable manually.

3. The control clamp as claimed in one of claims 1 or 2, characterized in that three clamping jaws (14, 14', 14'') are provided between which the hose (10) is clamped.

4. The control clamp as claimed in one of the preceding claims, characterized in that the clamping jaws (14, 14', 14'') are at least approximately of circular sector shape in cross section perpendicular to the longitudinal axis (20) of the hose (10).

5. The control clamp as claimed in one of the preceding claims, characterized in that the clamping jaws (14, 14', 14'') have the same configuration.

6. The control clamp as claimed in one of the preceding claims, characterized in that the clamping jaws (14, 14', 14'') are displaceable along radii (16, 16', 16'') which intersect the longitudinal axis (20) of the hose (10).

7. The control clamp as claimed in claim 6, characterized in that the edges (15, 15', 15'') of the clamping jaws (14, 14', 14'') engaging the hose (10) lie on the radii (16, 16', 16'').

8. The control clamp as claimed in claim 7, characterized in that there are free spaces (24, 24', 24''), laterally with respect to the direction of movement, between the clamping jaws (14, 14', 14'') as they are being displaced to reduce the flow cross section (12) of the hose (10), the hose being deformable in those free spaces such that the walls of the hose largely abut each other in parallel planes and the flow cross section at least approximately has the shape of a three-point star.

9. The control clamp as claimed in one of the preceding claims, characterized in that the clamping jaws (14, 14', 14'') clamp the hose (10) at least approximately in parallel for a distance of from 1 to 50 mm.

10. The control clamp as claimed in one of the preceding claims together with a hose, characterized in that the hose (10) is preshaped plastically at least in the range of the control clamp such that it can be clamped symmetrically between the clamping jaws (14, 14', 14'').

11. The control clamp as claimed in one of the preceding claims, characterized in that the outer surfaces (22, 22', 22'') of the clamping jaws (14, 14', 14'') lie at least approximately on the periphery of a cylinder or cone and are adapted to be pressed together by means of a sleeve (28) which embraces the clamping jaws.

12. The control clamp as claimed in claim 11, characterized in that the clamping jaws (14, 14', 14'') are provided with an external thread and the sleeve (28) is provided with an internal thread, the sleeve (28) and the clamping jaws (14, 14', 14'') being shaped conically in opposite directions.

13. The control clamp as claimed in one of claims 1 to 10, characterized in that the clamping jaws (14, 14', 14'') are adapted to be pressed together by means of a hose clamp.

14. The control clamp as claimed in one of the preceding claims, characterized in that a centering insert (26) for the hose (10) is provided between the clamping jaws (14, 14', 14'') to assure the central symmetric guidance of the hose with respect to the clamping jaws.

15. The control clamp as claimed in one of the preceding claims, characterized in that a connecting link guide means is provided for displacing the clamping jaws (14, 14', 14'').

Fig. 1

Fig. 2